Europäisches Patentamt

European Patent Office

Office européen des brevets

(11) Numéro de publication: **0 214 065**
**A1**

## (12) DEMANDE DE BREVET EUROPEEN

(21) Numéro de dépôt: 86420182.7

(22) Date de dépôt: 07.07.86

(51) Int. Cl.⁴: **A 61 F 2/32**
**A 61 F 2/36**

(30) Priorité: 09.07.85 FR 8510689

(43) Date de publication de la demande:
11.03.87 Bulletin 87/11

(84) Etats contractants désignés:
AT BE CH DE GB IT LI LU NL SE

(71) Demandeur: Brunet, Jean-Louis
Clinique Charcot 51-53 rue du Commandant Charcot
F-69110 Sainte-Foy-les-Lyon(FR)

(71) Demandeur: Brunet, André
14, allée de Bellevue
F-28130 Maintenon(FR)

(71) Demandeur: SOCIETE NOUVELLE DE ROULEMENTS
S.A.
Boîte Postale 17 1, rue des Usines
F-74010 Annecy Cedex(FR)

(72) Inventeur: Brunet, André
14, Allée de Bellevue
F-28130 Maintenon(FR)

(72) Inventeur: Sonnerat, Claude
1 Rue des Usines
F-74010 Annecy Cedex(FR)

(54) Prothèse de la hanche perfectionnée.

(57) L'invention a pour objet une prothèse de la hanche perfectionnée dans laquelle la prothèse fémorale oscille autour d'un axe incliné à 20° sur l'horizontale, caractérisée par le fait que cette oscillation est assurée à l'intérieur de la tête de la prothèse fémorale (1) par un roulement double comportant une rangée de rouleaux (2) pour supporter les charges radiales et une rangée de billes (3) pour supporter les charges axiales.

EP 0 214 065 A1

./...

FIG 1

1

0214065

Prothèse de la hanche perfectionnée

La présente invention due à la collaboration de Monsieur André BRUNET et de Monsieur Claude SONNERAT au nom de la SOCIETE NOUVELLE DE ROULEMENTS, se rapporte à une prothèse de la hanche perfectionnée.

Les prothèses totales de la hanche pratiquées habituellement sont le siège de frottements entre la partie sphérique remplaçant la tête du fémur et son logement implanté dans le bassin du patient. Du fait de ces frottements et quels que soient les matériaux utilisés, une usure se produit nécessitant le remplacement de tout ou partie de la prothèse au bout d'un certain temps.

Pour remédier à cet inconvénient, le brevet français 2.261.743 décrit une prothèse dont l'axe d'oscillation monté sur roulement est incliné à 20° par rapport à l'horizontale, cette disposition permettant lors de la marche de n'avoir qu'un seul mouvement d'oscillation autour de cet axe et donc de supprimer l'usure entre la tête sphérique et son logement puisque leur mouvement relatif devient alors nul.

Cependant, si l'on a supprimé le frottement entre la tête sphérique et son logement, le problème a été reporté au niveau du roulement qui est composé d'un axe tourillonnant dans un logement cylindrique et d'une rangée de billes permettant la tenue axiale de l'ensemble.

Lors du fonctionnement de la prothèse, une usure par frottement va se développer entre l'axe et son logement obligeant, à terme, une nouvelle intervention

La présente invention a pour objet une prothèse de la hanche perfectionnée éliminant totalement les inconvénients précités.

Essentiellement à cet effet, la prothèse de la hanche perfectionnée

2

selon l'invention, dans laquelle la prothèse fémorale oscille autour d'un axe incliné sensiblement à 20° sur l'horizontale, est caractérisée par le fait que cette oscillation est assurée à l'intérieur de la tête de la prothèse fémorale par un roulement double comportant une rangée de rouleaux pour supporter les charges radiales et, une rangée de billes pour supporter les charges axiales, de telle sorte que tout mouvement de frottement et donc d'usure soit supprimé.

D'autres caractéristiques et particularités d'une prothèse de la hanche suivant l'invention, apparaîtront à titre d'exemple en référence au dessin annexé dans lequel :

- la figure 1 est une vue en coupe axiale d'une prothèse de la hanche selon l'invention, vue en position de montage.

- la figure 2 est une vue en coupe axiale de la tête de la prothèse selon un premier mode de réalisation.

- la figure 3 est une vue en coupe axiale de la tête de la prothèse selon une variante de réalisation.

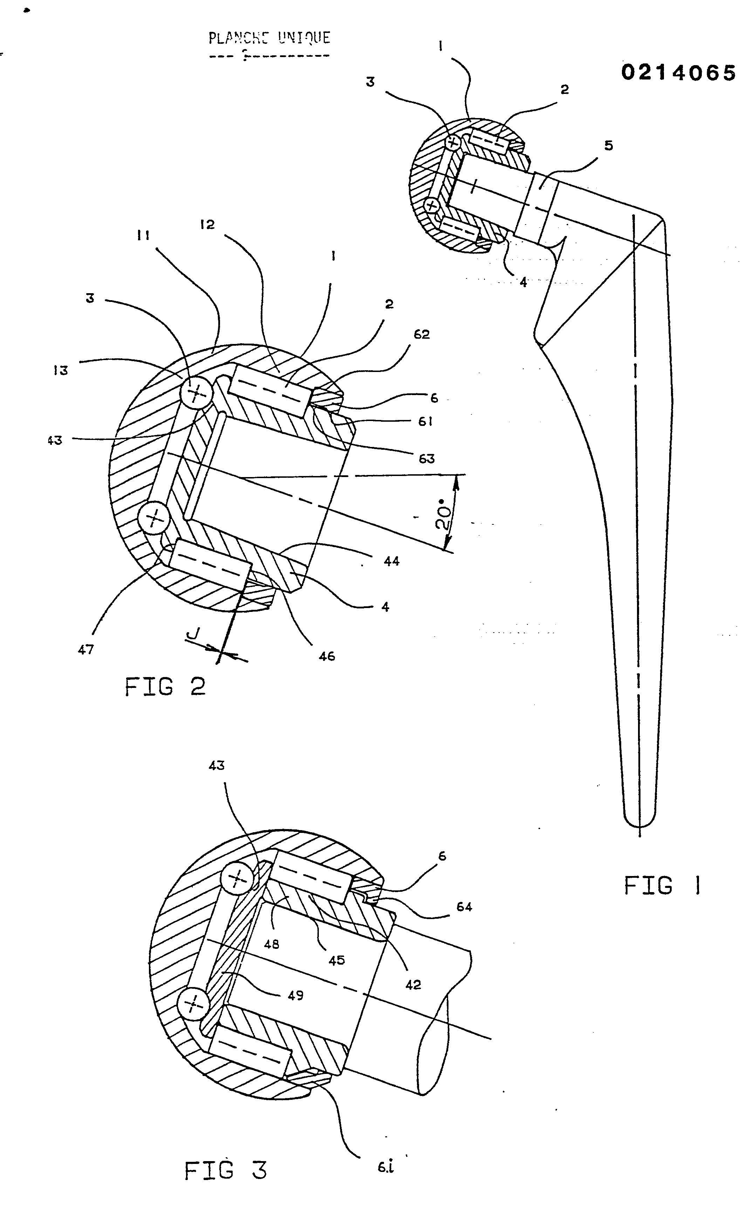

La prothèse de la hanche représentée à la figure 1 comporte essentiellement une tête sphérique 1 emmanchée sur le support fémoral 5 lequel est implanté dans le fémur du patient.

La tête sphérique 1 vient rotuler à l'intérieur d'un logement (non représenté) implanté dans le bassin du patient.

Selon l'invention, la tête de la prothèse 1 comporte un roulement à une rangée de rouleaux cylindriques (2) et un roulement à une rangée de billes 3, permettant la rotation sans frottement entre la bague extérieure 11 et la bague intérieure 4 de la tête de la prothèse 1, la bague intérieure 4 étant rendue solidaire du support

.fémoral 5 par un assemblage à emmanchement conique 44 (fig. 2) ou cylindrique 45 (fig. 3), ou tout autre emmanchement connu pour de tels assemblages (trigonal, cannelures, etc ...)

La bague extérieure sphérique 11 de la tête de la prothèse 1 comporte dans son alésage un chemin de roulement cylindrique 12 dans lequel viennent rouler les rouleaux 2 pour supporter les charges radiales, et un chemin de roulement torique 13 en.position sensiblement axiale dans le fond de son alésage dans lequel viennent rouler les billes 3 pour supporter les charges axiales qui viennent solliciter la prothèse pendant la marche.

De la même manière, la bague intérieure 4 possède sur son diamètre extérieur un chemin de roulement cylindrique 42 pour recevoir la rangée de rouleaux 2 et, sur sa face, en regard avec le fond de l'alésage de la bague extérieure, un chemin de roulement torique 43 pour recevoir la rangée de billes 3. Des épaulements 46 et 47 sont ménagés de chaque côté du chemin de roulement 42 afin d'assurer le guidage axial des rouleaux 2.

Une bague cylindrique 6 vient s'emmancher par son diamètre extérieur 62 dans un logement approprié de la bague extérieure 11 consécutif au chemin de roulement 12, afin de rendre indémontable l'ensemble des éléments 11, 2, 3, 4 constitutifs de la tête de la prothèse 1. Cette disposition est rendue possible par le fait que la bague 6 est soit frettée, soit collée dans son logement et, que son alésage 63 est inférieur au diamètre du chemin 12 et permet ainsi une retenue axiale des rouleaux 2. Un jeu J est menagé entre les rouleaux 2 et la face de la bague 6, afin d'éviter tout frottement pendant le fonctionnement de la prothèse.

Une étanchéité permettant d'éviter la pollution des roulements par des particules est assurée par l'intermédiaire de l'alésage 61 de la bague cylindrique 6 qui vient en regard du diamètre extérieur de la bague intérieure 4 en ménageant entre ces deux pièces un jeu minimal de fonctionnement.

Selon un autre mode de réalisation, la bague cylindrique 6 est directement emmanchée dans le prolongement du chemin de roulement 12 comme indiqué sur la figure 3.

Les deux rangées de roulements 2 et 3 peuvent être comme représenté avec des éléments (rouleaux ou billes) de roulements jointifs, ou de façon connue en soi, posséder des cages métalliques ou plastiques de séparation des corps roulants.

La bague intérieure 4 pourra être soit monobloc comme représenté à la figure 2, soit en deux parties 48 et 49 comme représenté à la figure 3, pour permettre une meilleure répartition des charges sur chacune des deux rangées de roulements. Dans ce cas, la bague 6 pourra par exemple comporter une collerette radiale 64 coopérant avec un embrèvement de forme appropriée de la demi-bague 48 pour permettre le maintien des différentes pièces constitutives de la tête de la prothèse pendant les opérations de montage.

Bien entendu, d'autres variantes de réalisation peuvent encore être imaginées sans pour autant sortir du domaine de l'invention.

REVENDICATIONS

1 Prothèse de la hanche perfectionnée , caractérisée par le fait que la prothèse fémorale oscille autour d'un seul axe incliné sensiblement à 20° sur l'horizontale , cette oscillation étant assurée , à l'intérieur de la tête de la prothèse fémorale ( 1 ) par un roulement double comportant une rangée de roulements ( 2 ) pour supporter les charges radiales , et une rangée de roulements ( 3 ) pour supporter les charges axiales . chacune de ces rangées utilisant un roulement sans frottement pour éviter toute usure .

2 Prothèse de la hanche selon la revendication 1 , caractérisée par le fait que la rangée de roulements ( 2 ) supportant les charges radiales est un roulement à rouleaux cylindriques

3 Prothèse de la hanche selon la revendication 1 , caractérisée par le fait que la rangée de roulements ( 3 ) supportant les charges axiales est une butée à billes.

4 Prothèse de la hanche selon les revendications 1 à 3 , caractérisée par le fait que la fixation de la bague intérieure de la tête fémorale ( 1 ) sur le support fémoral ( 5 ) est réalisée au moyen d'un assemblage à emmanchement conique ( 44 ).

5 Prothèse de la hanche selon les revendications 1 à 4 , caractérisée par le fait que les rouleaux ( 2 ) sont guidés sur la bague intérieure ( 4 ) par l'intermédiaire de 2 épaulements ( 46 - 47 ).

6 Prothèse de la hanche selon les revendications 1 à 5 , caractérisée par le fait que la bague intérieure ( 4 ) et la bague extérieure ( 11 ) constituant la tête fémorale ( 1 ) sont rendues indissociables par l'intermédiaire d'une bague ( 6 ) emmanchée dans un logement correspondant de la bague extérieure et venant retenir l'ensemble de la nappe des rouleaux ( 2 ) tout en assurant un étanchéité ( 61 ) avec le diamètre extérieur de la bague intérieure ( 4 ).

7 Prothèse de la hanche selon les revendications 1 à 6 , caractérisée par le fait que la bague intérieure ( 4 ) est en deux parties ( 48 et 49 ) , la première demi-bague ( 48 ) comportant le chemin de roulement à rouleaux ( 42 ) , tandis que l'autre demi-bague ( 49 ) comporte le chemin de roulement à billes. .

0214065

FIG 2

FIG 3

FIG 1

Office européen
des brevets

**RAPPORT DE RECHERCHE EUROPEENNE**

**0214065**
Numero de la demande

EP 86 42 0182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | FR-A-2 357 235 (LEVEAU et al.)<br>* Page 1, lignes 19-29; page 3, lignes 4-6; figures * | 1,2 | A 61 F 2/32<br>A 61 F 2/36 |
| A | | 5 | |
| A | FR-A-2 048 698 (OSTEO)<br>* Page 2, lignes 6-10; figure * | 1,3,6 | |
| A | US-A-3 683 421 (MARTINIE)<br>* Colonne 6, lignes 5-8; figures 7,8 * | 1,2,4 | |
| A | FR-A-2 060 179 (CELLIER)<br>* Page 2, lignes 35-39; page 3, lignes 18-26; figures * | 1,3,6 | |
| A,D | FR-A-2 261 743 (BRUNET)<br>* Page 2, lignes 6-10; figures * | 1 | A 61 F |

DOMAINES TECHNIQUES
RECHERCHES (Int Cl 4)

A 61 F

Le present rapport de recherche a ete etabli pour toutes les revendications

| Lieu de la recherche | Date d'achevement de la recherche | Examinateur |
|---|---|---|
| LA HAYE | 15-10-1986 | GLAS J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet anterieur, mais publié à la date de dépôt ou apres cette date
D : cité dans la demande
L : cité pour d'autres raisons

& : membre de la même famille, document correspondant